# EUROPEAN PATENT SPECIFICATION

(11) **EP 4 436 441 B1**
(45) Date of publication and mention of the grant of the patent: **14.01.2026**
(21) Application number: 22817654.1
(22) Date of filing: 14.11.2022
(51) Int. Cl.: A46B 15/00, A61B 5/11, A61C 17/22, A61B 5/00

(54) **ORAL CARE DEVICE WITH TOOTH MOBILITY DETECTION**
MUNDPFLEGEVORRICHTUNG MIT ERKENNUNG DER ZAHNBEWEGLICHKEIT
DISPOSITIF DE SOIN BUCCAL AVEC DÉTECTION DE LA MOBILITÉ DES DENTS

(30) Priority: 22.11.2021 US 202163281875 P; 28.04.2022 EP 22170456
(43) Date of publication of application: 02.10.2024
(73) Proprietor: Koninklijke Philips N.V., 5656 AG Eindhoven (NL)
(72) Inventor: BRANDAO SILVA, Priscilla, 5656 AG Eindhoven (NL); GERHARDT, Lutz Christian, 5656 AG Eindhoven (NL); JOHNSON, Mark Thomas, 5656 AG Eindhoven (NL); KOOIJMAN, Gerben, 5656 AG Eindhoven (NL)
(74) Representative: Philips Intellectual Property & Standards
(86) International application number: PCT/EP2022/081703
(87) International publication number: WO 2023/088814

(56) References cited:
- CN-A- 112 107 386
- US-A- 5 518 008

## Description

### FIELD OF THE INVENTION

The present application relates to an oral care device, particularly an oral cleaning device.

### BACKGROUND OF THE INVENTION

Mobility of teeth is a valuable clinical marker within oral care.

For example, knowledge of tooth mobility (TM) is important in connection with monitoring periodontitis disease progression, bone loss as well as for monitoring periodontal or orthodontic therapy response and therapy efficacy. Increased tooth mobility is a common symptom of periodontal disease and is one of the main reasons for a visit to a dental physician (DP).

Teeth are fixed in jawbone sockets by a periodontal ligament. The periodontal ligament not only provides firm binding support to teeth but also allows slight movement of teeth during chewing. This physiological mobility enables teeth to withstand a significant force during chewing.

Normal (physiological) tooth mobility varies between different teeth and ranges for example from 40 µm (for molars) to 120 µm (for incisors). However, certain pathological conditions of the bone or periodontal ligament can adversely affect the teeth mobility. Increased tooth mobility is a gradual and irreversible process. If not corrected in time this can also lead to loss of a tooth.

For example, 50% of Americans aged 30 or older (65 million) have periodontitis. In studies, 10-20 % of periodontitis patients showed tooth mobility of some degree and at multiple tooth sites. Increased tooth mobility can also be caused by orthodontic treatment, malocclusion issues, and bruxism.

A clinical decision as to whether tooth mobility requires a treatment response is based on detected severity or degree of tooth mobility.

The existing clinical methods for tooth mobility assessment are subjective, time consuming and error prone. Researchers have proposed other methods such as use of laser beams, or the Peritotest device (a handheld device with an electric plunger, which touches a tooth or implant in rapid succession to determine its stability). However, these are complex and can only be used by a professional dental practitioner.

Within the state of the art, no system is available to provide tooth mobility measurement and diagnosis in a simple manner, which might also be performed at home.

CN112107386A provides a system for acquiring and processing user health data through smart oral care products and mobile devices, which collect and transmit the data to generate a user representation.

### SUMMARY OF THE INVENTION

The invention is defined by the claims.

According to examples in accordance with an aspect of the invention, there is provided an oral care device, comprising:
a support body;
a plurality of cleaning elements for mechanical engagement with oral surfaces, the cleaning elements protruding from a surface of the support body;
a sensor means adapted to sense a measure indicative of a loading force/pressure applied to, or motion of, at least one of the cleaning elements; and
a processing arrangement comprising one or more processors adapted to:
   receive a sensing signal from the sensor means corresponding to a period of mechanical engagement of the at least one cleaning element with at least one tooth during an oral cleaning session,
   process a waveform of the signal to extract one or more characteristics of the signal waveform for the time period;
   determine a measure indicative of a degree of tooth mobility for the at least one tooth; and
   generate a data signal indicative of the determined measure indicative of a degree of tooth mobility, for output from the device.

Thus embodiments propose incorporating force, pressure, or motion sensing in a home-based oral care device in order to detect a degree or level of tooth mobility during the course of normal use of the oral care device for its oral care function. For example, the tooth mobility can be assessed from sensor measurements acquired during an oral cleaning session of an oral cleaning device such as a toothbrush or mouthpiece device. In this way, tooth mobility can be assessed at home, in a convenient manner.

Determining the degree of tooth mobility may comprise performing a spectral analysis of the sensing signal.

In particular, the oral care device may include an actuation means for driving an oscillatory motion of the at least one cleaning element with an oscillatory motion pattern having a frequency spectrum. Tooth mobility affects the oscillatory motion pattern of the cleaning elements in a manner that is related in a reliable way to the degree of tooth mobility. This factor can thus be used to detect the tooth mobility according to at least one set of embodiments.

In some embodiments, determining the degree of tooth mobility may comprise computing a frequency spectrum for the sensing signal. It may further comprise detecting an amplitude of one or more frequency components of the sensing signal. It may comprise detecting an amplitude of a central frequency of at least a main or dominant peak in the frequency spectrum (i.e. the highest peak). Additionally or alternatively, it may further comprise detecting a bandwidth associated with the aforementioned main or dominant peak of the frequency spectrum or a quality factor associated with the main or dominant peak of the frequency spectrum. It may comprise comparing any of these characteristics with a reference or baseline. It may comprise processing any of these characteristics with a pre-determined transfer function or lookup table to derive the degree of tooth mobility. It may comprise monitoring changes in any of these characteristics for a same user over one or a plurality of cleaning sessions.

As mentioned above, the oral care device may in some embodiments include an actuation means for driving an oscillatory motion of the at least one cleaning element which, in a reference or calibration condition, exhibits an oscillatory motion pattern having a reference frequency spectrum which includes a main or dominant peak, and the peak having a central frequency component. Determining the degree of tooth mobility may comprise determining changes between the reference frequency spectrum and the sensed frequency spectrum of one or more of: an amplitude of a central frequency of the main or dominant peak of the frequency spectrum; a bandwidth associated with a main or dominant peak of the frequency spectrum; and/or a quality factor associated with a main or dominant peak of the frequency spectrum.

In other words, the oscillatory motion has a spectral signature, and wherein determining the metric of tooth mobility comprises detecting changes in said spectral signature. However it is not essential to compare the sensed frequency spectrum, or characteristics thereof, with a reference frequency spectrum. The frequency signal characteristics may simply be measured and used in combination for example with a transfer function to derive the degree of tooth mobility.

The reference frequency spectrum referred to above could for instance correspond to an expected motion frequency spectrum for a controlled calibration condition, for instance for a reference load, and for a certain reference tooth (e.g. a non-mobile tooth), e.g. in a brushing session after a dental examination, or against a well-defined surface using a standardized method.

The reference frequency spectrum could be stored as calibration data in a memory of the processing arrangement.

There could be stored multiple different reference frequency spectra corresponding to different possible applied user loads.

There are different options for the sensor means.

In some embodiments, the sensor means is for sensing force or pressure exerted on the at least one cleaning element.

In some embodiments, the processor may be adapted to perform a calibration operation comprising sensing a baseline or average loading force applied to the at least one cleaning element during one cleaning session, or over a plurality of cleaning sessions, and wherein the degree of tooth mobility is determined in dependence upon the baseline or average loading force.

For example, the metric of tooth mobility may be based on a measured change in the amplitude or the width of the dominant peak (i.e. damping) in the frequency spectrum of the oscillation of the cleaning element, and wherein the baseline loading force is taken into account in this computation, e.g. to normalize the detected amplitude or damping change.

The damping is proportional to the width of the resonant peak about the peak's center frequency.

**In** some embodiments, the oral care device may be a toothbrush. The toothbrush may comprise a brush head coupled to a main body. The main body may form a handle for the device. The support body may be a platen of the brush head. The cleaning elements may comprise bristles.

The sensing means may comprise a force or pressure sensor adapted to detect loading force applied to the brush head relative to the main body.

For example, the sensing means may be adapted to detect pivotal forces applied to the head relative to the body.

The sensing means may be incorporated in the head or may be incorporated in the main body, or may have components incorporated in both parts.

Additionally or alternatively, the sensor means may comprise a pressure or force sensor comprised by the support body, arranged to detect loading forces applied to the at least one cleaning element. For example the sensor means may be arranged for mechanical communication with the at least one cleaning element,

For example, in at least one set of embodiments, the sensor means may comprise a pressure-sensitive ring member extending around a base of the at least one cleaning element, for detecting loading pressure applied to the cleaning element in at least one direction perpendicular to a direction of extension of the cleaning element.

The pressure-sensitive ring member may comprise a pressure-responsive electroactive material, e.g. a piezoresistive material or an electroactive polymer.

In some embodiments, the oral care device may be a mouthpiece device. The support body may be for receipt inside the oral cavity. The support body may have an arcuate shape for extending around at least a portion of a user's dental arch.

The mouthpiece device may comprise at least one tooth-receiving channel.

The cleaning elements may protrude into the tooth-receiving channel.

In some embodiments, the sensing means may comprise one or more force or pressure sensors integrated in at least one wall of the tooth-receiving channel.

Additionally or alternatively, the cleaning elements may extend into the tooth receiving channel for engagement with teeth, and wherein the at least one cleaning element comprises a pressure-responsive material for sensing force or pressure applied to the at least one cleaning element, and wherein the pressure-responsive material forms at least a part of the sensor means.

In some embodiments, the support body of the mouthpiece device may comprise an arcuate body for following the contour of only one portion of the user's dental arch, and wherein the body is moveable by a user from one side of the mouth to a second side of the mouth, and wherein the processor is adapted to acquire at least one tooth mobility metric for each side of the mouth. This configuration may be referred to as a J-shaped mouthpiece.

The processor may compare the results from the left and right sides, or top and bottom rows, to provide a degree of localization of tooth mobility detection.

User-implemented movement from one side to the other may be detected with an inertial sensor integrated in the body in some embodiments. For example, movement to the other side may involve inverting or flipping the body, which motion can be detected.

In some embodiments, the support body may comprise a plurality of sections, and each comprising a respective sensor for sensing force on, or motion of, at least one cleaning element protruding from the respective section, and wherein a respective tooth mobility metric is determined for each section.

The sections may be linked together, e.g. in an articulated structure.

In some embodiments, each section of the body may be vibrationally insulated from each neighboring section. Additionally or alternatively, each section of the body may be independently drivable with an oscillatory motion.

In any of the embodiments described hereinabove, the sensing means may be adapted to detect loading forces applied to, or motion of, the at least one cleaning element in one or more of: a direction normal to the surface of the support body; and one or more directions parallel with a surface of the support body.

The sensing means may be adapted to detect loading force or pressure applied to, or motion of, the at least one cleaning element in three orthogonal directions.

In some embodiments, the processing arrangement is further adapted to determine a degree of tooth mobility for each of a plurality of teeth in the oral cavity over the course of an oral cleaning session, to thereby generate a tooth mobility data record for the oral cleaning session. The processing arrangement may be further adapted to transfer the tooth mobility data record to a datastore, tagged with a time-stamp, and optionally tagged with user identification information.

The datastore may be local to the device, or may be remote from the device, for example a cloud-based datastore with the physical storage means located at a remote server.

A further aspect of the invention provides a system comprising the oral care device in accordance with any embodiment described in this disclosure, or any claim of this application, and comprising one or more further components.

The system may further comprise a datastore for storing previously determined metrics of tooth mobility for one or more subjects.

The system may further comprise a secondary device comprising a processing arrangement adapted to: access the datastore, and retrieve one or more tooth mobility data records for a user. The secondary device may be adapted to retrieve multiple tooth mobility data records for a given user for a series of different time points, each record comprising tooth mobility metrics for one or more teeth; and control a user interface of the secondary device to display trend information indicative of the one or more tooth mobility metrics as a function of time.

The secondary device may for example comprise a mobile computing device such as a smartphone.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an example oral care device in accordance with one or more embodiments;
Fig. 2 illustrates a block diagram of components of an example oral care device in accordance with one or more embodiments;
Figs. 3 and 4 illustrate a sensing principle for sensing force on or motion of cleaning elements of an oral care device;
Fig. 5 illustrates an example sensing signal waveform;
Fig. 6 illustrates examples frequency spectra of a segment of a sensing signal waveform;
Fig. 7 illustrates an example oral care device according to some embodiments;
Fig. 8 illustrates a further example oral care device according to some embodiments;
Fig. 9 illustrates an example sensor means in the form of sensitive ring elements for surrounding cleaning element tufts;
Fig. 10 illustrates a relationship between applied user load and the measured sensing signal response;
Fig. 11 illustrates an example oral care device in the form of a mouthpiece device;
Fig. 12 further illustrates an example oral care device in the form of a mouthpiece device;
Fig. 13 illustrates example mouthpiece devices having a sensor means integrated in walls of one or more tooth receiving channels; and
Fig. 14 illustrates example mouthpiece devices having electroactive cleaning elements for performing a sensing function.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an oral care device having integrated means for detecting a level of tooth mobility. A sensor means is included which outputs a sensing signal indicative of force or pressure applied to, or motion of, cleaning elements of the device during a cleaning session. By processing a waveform of this signal to detect one or more signal characteristics, a measure indicative of a level or degree of tooth mobility can be derived.

For example, certain embodiments propose using measurements of variations in force, pressure, or acceleration signals pertaining to dynamics of an oral health care device functional component which makes contact with teeth during use (such as cleaning elements of a powered toothbrush or cleaning mouthpiece). Variations in such signals can be expected in dependence upon a degree of tooth mobility of a tooth which is contacted. The signals may change therefore as the oral care device is moved across the teeth during a cleaning session (e.g. during brushing). A processing arrangement can be provided adapted to convert the measured signal (e.g. using an algorithm or transfer function) into a tooth displacement measure or a measure indicative of a level of tooth mobility.

The processing arrangement may also monitor the variation of characteristic signal parameters (amplitude, damping) derived from force/pressure/acceleration signals measured over multiple oral care sessions, for example for use in monitoring progression or onset of tooth mobility.

Certain embodiments may include functionality, such as one or more algorithms, for performing triaging and/or automated scheduling of clinical appointments based on determined tooth mobility levels.

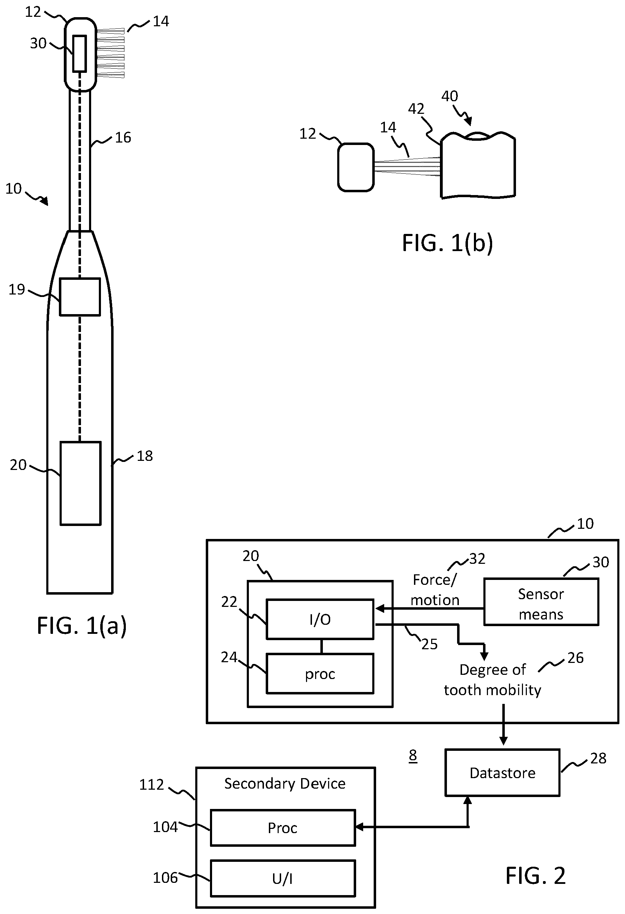

Fig. 1 and 2 illustrate an oral care device 10 which is in accordance with at least one set of embodiments of the invention The device 10 comprises a support body 12, from at least one surface of which protrude a plurality of cleaning elements 14 for mechanical engagement with oral surfaces 42. The oral surfaces 42 may be surfaces of teeth 40 or for instance of dental implants. The device further includes a sensor means 30 adapted to sense a measure indicative of a loading force or pressure applied to, or motion of, at least one of the cleaning elements 14 or the support body.

The device further comprise a processing arrangement 20.

The processing arrangement comprises an input/output (I/O) 22 and one or more processors 24 adapted to: receive at the I/O 22 a sensing signal 32 from the sensor means 30 corresponding to a period of mechanical engagement of the at least one cleaning element 14 with at least one tooth 40 during an oral cleaning session; process a waveform of the signal 32 to extract one or more characteristics of the signal waveform for the time period; determine a measure indicative of a degree of tooth mobility 26 for the at least one tooth 40; and generate a data signal 25 indicative of the determined measure indicative of a degree of tooth mobility, for output from the device at the input/output 22.

Thus, embodiments propose detecting a measure correlated with degree or level of tooth mobility, for example based on a variation pattern of the sensing signal over said period of engagement with the at least one tooth, for instance as reflected in certain signal characteristics of a waveform of the sensing signal.

It is noted that the above-mentioned processing arrangement may also be provided as a separate element, independent of the other features of the oral care device.

With reference to Fig. 1, the oral care device in the illustrated embodiment is shown in the form of a toothbrush with a brush head 16 coupled to a main body 18. The main body forms a handle for the device. The support body 12 is provided by a platen of the brush head. The cleaning elements 14 may comprise bristles. The bristles may be arranged into tufts. The reference to at least one cleaning element above could be reference to at least one tuft of bristles in this set of embodiments.

Although bristles are mentioned as an example, the skilled person will be aware that other varieties of cleaning element are also possible, such as elastomeric cleaning elements (thicker than a typical individual bristle), or cleaning structures which protrude from the support body having extended shapes, but which are also designed to engage with oral surfaces to provide a mechanical cleaning action.

Although a toothbrush is shown in Fig. 1, in further examples, the oral care device could take a different form, such as a brushing mouthpiece device, or an oral irrigation device, or an oral flossing device.

The block diagram structure of Fig. 2 is applicable to an oral care device of any particular type, not just to a toothbrush.

With further reference to Fig. 1, preferably the oral care device includes an actuation means 19 for driving an oscillatory motion of the at least one cleaning element 14.

This induced oscillatory motion pattern of the at least one cleaning element exhibits a frequency spectrum. The device may records in a memory of the processing arrangement 20 (not shown) one or more reference frequency spectra which correspond to frequency spectra of the driven oscillatory motion pattern in certain reference or calibration conditions, such as in the presence of a certain reference loading force and to a certain tooth with a reference level of mobility (e.g. zero mobility). The reference condition could be any desired condition, e.g. in the absence of externally applied loading forces to the at least one cleaning element, and/or for a tooth with non-zero mobility. There could be more than one reference spectrum stored, for different conditions, e.g. different applied used loads (this is discussed further below). In all cases, the reference frequency spectrum has one or more dominant peaks, where the dominant peak means the peak with highest amplitude. The dominant peak has a central frequency component, and further has a bandwidth, and a quality factor associated with it. By comparing characteristics of a frequency spectrum of a sensed oscillatory motion pattern with characteristics of the reference oscillatory motion pattern, a level of tooth mobility may be detectable.

Each dominant peak may correspond to a harmonic of a dominant component of the applied oscillation pattern, i.e. is a resonance peak. Reference herein to a dominant peak means the peak with highest amplitude.

The sensor means 30 referred to above may take different forms, for example a pressure sensor, force sensor, inertial sensor (e.g. accelerometer), or load cell. The sensor means may be adapted to sense variations of acceleration, pressure, and/or force in the support body or the cleaning elements in one or more directions, e.g. normal to the support body surface from which the cleaning elements protrude, or preferably, in the three orthogonal axes.

The processing arrangement is adapted to convert the sensing loading signal variations into a tooth mobility level or grade identification.

With reference to Fig. 2, optionally in some embodiments, the processing arrangement 20 is further adapted to: determine a degree of tooth mobility 26 for each of a plurality of teeth 40 in the oral cavity over the course of an oral cleaning session, and to generate a tooth mobility data record for the oral cleaning session comprising the tooth mobility measures 26 for the plurality of teeth. The processing arrangement may further be adapted to transfer the tooth mobility data record to a datastore 28, tagged with a time-stamp, and optionally tagged with user identification information. An identification of the particular tooth to which each mobility measure corresponds may also be obtained (for instance using a positioning means), and this identification stored in association with the tooth mobility measure in the data record.

With reference to Fig. 2, another aspect of the invention may provide a system 8 comprising: an oral care device 10 in accordance with any embodiment described in this disclosure or in accordance with any claim of this application; and further comprising a datastore 28 for storing previously determined metrics of tooth mobility for one or more subjects.

With reference again to Fig. 2, optionally, in some embodiments, the system may further comprise a secondary device 112 comprising a processing arrangement 104 adapted to access the datastore 28 and to retrieve multiple tooth mobility data records for a given subject for one or more different time points.

Data records for a plurality of different time points may be retrieved, each record comprising tooth mobility measures for one or more teeth. The processor 104 of the secondary device 112 may generate by processing the retrieved data records trend information indicative of the one or more tooth mobility measures as a function of time. The processor 104 of the secondary device 112 may control a user interface 106 of the secondary device to display the trend information.

The secondary device may for example comprise a mobile computing device such as a smartphone.

In some embodiments, the processing arrangement 104 may be adapted to compare the one or more characteristics of the sensing signal waveform with corresponding characteristics for a stored baseline or reference sensing signal to determine the tooth mobility level. The reference signal may for example correspond to a signal for a certain reference tooth (e.g. permanent healthy tooth which could be indicated by a dental practitioner).

In some embodiments, an averaged sensing signal for multiple cleaning sessions may be computed by storing the sensing signal for each session in a memory and then retrieving the stored signals and computing the average. This average signal could be used for determining the level of tooth mobility instead of (or in addition to) the signal for just a single cleaning session.

In some embodiments, the measure of tooth mobility could be derived for a given tooth by comparing the one or more characteristics of the signal waveform for the given tooth with those of one or more other teeth acquired within the same cleaning session.

In at least one set of embodiments, it is proposed to determine tooth mobility using a spectral analysis of the sensing signal. For example, determining tooth mobility may comprise assessing, within the frequency domain of the sensing signal, variations of signal amplitude at one or more frequency points, and/or damping of a main harmonic component of the oscillation pattern over one or more cleaning sessions. The variations may be monitored over multiple cleaning sessions, or within the same cleaning session using differential measurement among different teeth.

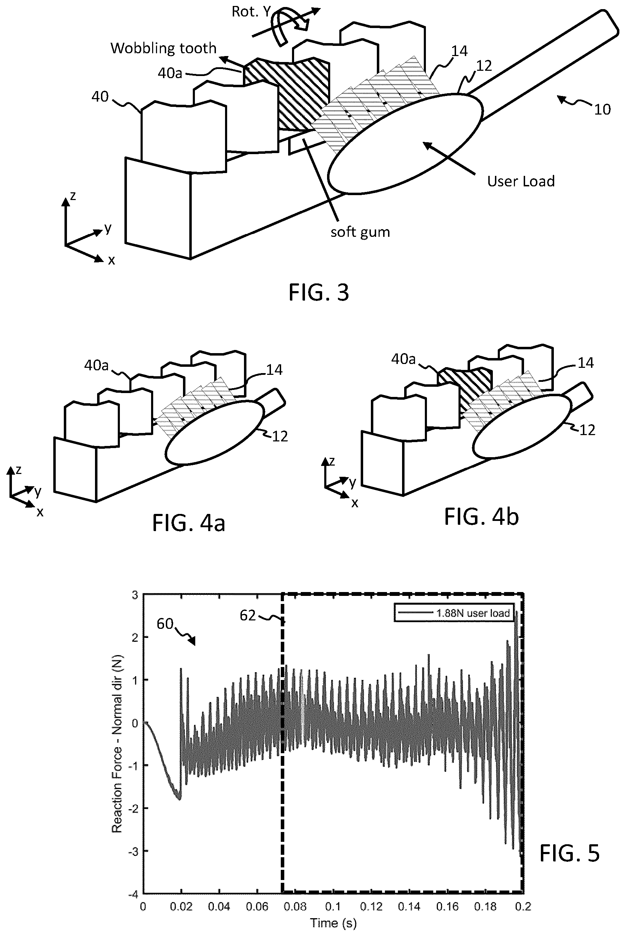

With reference to Fig. 3 and Fig. 4, the sensing principle is illustrated. Fig. 3 shows mechanical engagement of cleaning elements 14 of an example oral care device 10 with teeth 40. The user, in cleaning their teeth, applies the cleaning elements with a certain user load force, which includes a component normal to the surface of the tooth. As illustrated in Fig. 4, when the cleaning elements engage a tooth which has a high level of mobility ("wobbly tooth"), the tooth exhibits motion. The motion of the highlighted tooth 40a is predominantly motion about the y-axis, but also exhibits some smaller degree of motion about the x-axis.

Fig. 3 and Fig. 4 schematically represent the geometry and physical principles underlying computer simulations which were run to test and demonstrate the feasibility of detecting tooth mobility. The model employed an *in-silico* tooth-jaw model.

Fig. 4(a) represents a simulation in which the tooth of interest 40a demonstrates a low level of tooth mobility (the maximum tooth displacement being 0.05mm, representative of normal physiological tooth movement). Fig. 4(b) represents a simulation in which the tooth of interest is a wobbling tooth demonstrating a high level of tooth mobility (the maximum tooth displacement being 1.5mm, a large wobbling amplitude, typical of tooth mobility for a tooth that will subsequently be lost.

The in-silico model consisted of a jaw with soft gums around the reference tooth 40a, and wherein the reference tooth is free to rotate around the mesial-distal (Y) axis and is connected through a torsional spring to the root, as schematically in Fig. 3.

The level of mobility of the reference tooth is controlled in the model by tuning the stiffness coefficient of the torsional spring attached to it. Brushing conditions were simulated based on a Philips A3 brush head, with a 90° angle of the cleaning elements relative to the tooth surface (90° brushing angle) and a constant user load of 1.88N (average load among Philips toothbrush users). The motion pattern of the cleaning elements was modeled as a large-amplitude medial-distal stroke (applied by a user) superposed with a smaller amplitude oscillatory motion pattern induced by the actuation/drive mechanism of the powered toothbrush.

A simulated sensing signal representing reaction forces on the brush head platen 12 as a function of time, as well as the simulated tooth displacement, were computed from the model in the two mobility conditions of Fig. 4(a) and Fig. 4(b).

Fig. 5 shows a representation of a waveform 60 of the obtained simulated sensing signal in the time domain.

The received sensing signal waveform 60 may typically comprise signal segments corresponding to respective periods of engagement of the at least one cleaning element with each of a plurality of teeth. The processing arrangement 20 may be adapted to determine a respective metric of tooth mobility for each of the plurality of teeth. An identification of each of the teeth may further be obtained in some examples.

Within the simulated sensing signal result of Fig. 5, a signal segment 62 corresponding to the modelled tooth of interest 40a is indicated.

In accordance with at least one set of embodiments, determining the degree of tooth mobility may comprise performing a spectral analysis of the sensing signal.

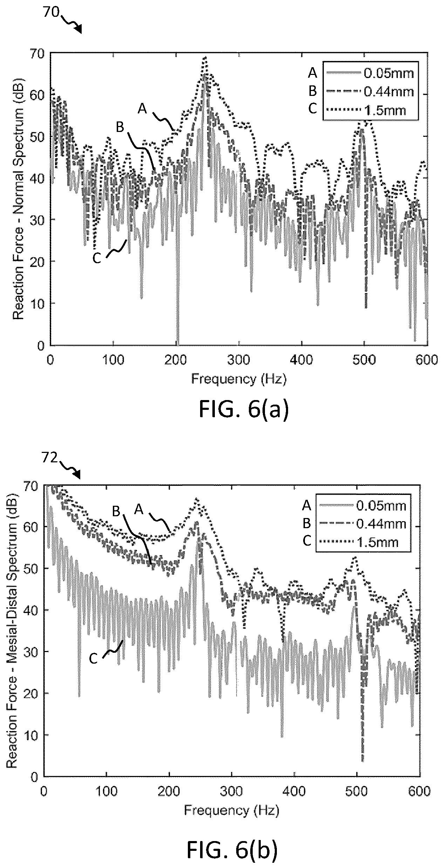

This is illustrated further with reference to Fig. 6.

To compute the measure indicative of a degree of tooth mobility, the processing arrangement in accordance with at least one set of embodiment is adapted to compute a frequency spectrum 70, 72 for the sensing signal.

The plots of Fig. 6 each show frequency spectra for each of three different tooth mobility levels, labelled A, B and C. Level A corresponds to a mobility amplitude of 0.05 mm. Level B corresponds to a mobility amplitude of 0.44 mm. Level C corresponds to a mobility amplitude of 1.5 mm.

Fig. 6(a) shows the frequency spectra 70 computed for the normal reaction force components (x-direction in Figs. 3-4) of the sensing signal at each of the three different tooth mobility levels, A, B, C.

Fig. 6(b) shows the frequency spectra 72 computed for the mesial-distal reaction force components (y-direction in Figs. 3-4) of the sensing signal at each of the three different tooth mobility levels, A, B, C.

As mentioned above, in this, the oral care device is one which includes an actuation means 19 for driving an oscillatory motion of the at least one cleaning element 14 which, exhibits a baseline/reference frequency spectrum, and where this frequency spectrum has a main or dominant peak within it. This main or dominant peak corresponds to a main harmonic component of the applied oscillation pattern.

Observing the frequency domain plots 72, 74 for the reaction forces, there is a large increase in the reaction force amplitude levels for frequency components around the harmonics of the main vibration frequency (around 250 Hz) of the reference frequency spectrum of the actuation means. These show as dominant peaks in the frequency spectrum. There is also an increase in the damping level between frequency components around these dominant peaks. In particular, the amplitude of the frequency component corresponding to the main harmonic component of the oscillatory driving force applied by the actuation means (at around 250 Hz) shows an amplitude increase of about 4dB (in the normal direction) and 9.6dB (in the mesial-distal direction) from the lowest to the highest level of tooth mobility simulated.

The changes in the damping around the harmonic frequency components are qualitatively observable by the changes in bandwidths, and could be determined quantitatively by, for instance, computing the quality factor (Q factor), i.e. a dimensionless parameter defined as the ratio of the resonance center frequency to its bandwidth. For example, for a -3dB bandwidth, the Q factor for the main harmonic component may range from 30 in the case of a low level of mobility (case A) to 25 a high mobility case (case C) for the normal force, and, similarly for the mesial-distal force, it reduces from 31 to 25.7.

Thus, following this approach, in accordance with at least one set of embodiments, the determining the degree of tooth mobility may comprise detecting an amplitude of one or more frequency components of the sensing signal.

In some examples, determining the degree of tooth mobility may be performed by determining changes between the frequency spectrum of the oscillation applied by the actuation means and the sensed frequency spectrum of an amplitude of a central frequency of a dominant peak in the applied oscillation frequency spectrum.

Additionally or alternatively, in some examples, determining the degree of tooth mobility may be performed by determining changes between the frequency spectrum of the oscillation applied by the actuation means and the sensed frequency spectrum of a bandwidth associated with a dominant peak in the frequency spectrum or a quality factor associated with a dominant peak of the frequency spectrum.

There are different options for the sensor means 30 used to sense the force, pressure or displacement at the cleaning elements.

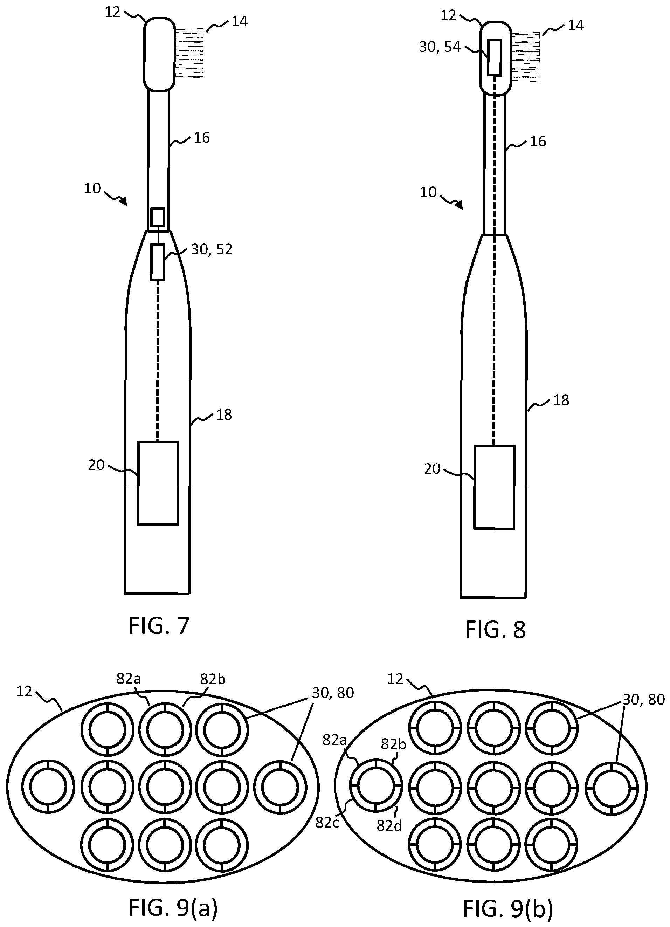

Fig. 7 schematically illustrates an example in accordance with at least one set of embodiments.

With reference to Fig. 7, in this example, the sensor means 30 takes the form of a force, pressure or inertial sensor (e.g. IMU) 52 provided integrated in the oral cleaning device main body portion 18 (which forms a handle for the device). The handle may be weakly coupled to the brush head 16, and variations in brush head motion dynamics/pattern can be monitored by measuring the relative motion/forces between the brush head 16 and the handle. In further examples, in case of strong coupling and low damping between the brush head and main body 18, mechanical coupling means may be provided to couple the vibration pattern of the brush head 16 to the sensor 52 in the main body, for example a mechanical element like a shaft or rod.

In either case, the sensor 52 is able to detect dynamical signal variations caused by tooth mobility. Results from simulations show that brush head platen dynamics (reaction force signal) is sensitive to small variations in tooth mobility.

A processing arrangement 20 providing signal acquisition and processing functions may be located in the handle 18, and electrical connections provided between the sensor 52 and the processing arrangement.

Thus, in summary, in this embodiment, the sensing means 30 comprises a force or pressure sensor 52 adapted to detect a loading force applied to the brush head 16 relative to the main body 18.

Fig. 8 schematically illustrates a further example in accordance with at least one set of embodiments.

With reference to Fig. 8, in this example, a sensor means 30 is provided in the form of a force, pressure or motion sensor integrated in the support body 12. In this example, the oral care device is in the form of a toothbrush and the support body is formed by a platen of the brush head 16. The sensor detects motion and/or force applied to the cleaning elements 14 for instance via detection of motion of the support body 12 (e.g. inertial sensing), or detection of force/pressure exerted on the support body surface, or exerted upon the cleaning elements themselves. In the latter case the sensing means may include a pressure or force sensor 54 comprised by the support body and arranged for mechanical communication with at least one cleaning element, to detect loading forces applied to the at least one cleaning element

Variations in the dynamics (i.e. acceleration, force in a single (normal) or multiple directions) of the brush head platen, or cleaning elements, are associated with differing tooth mobility levels.

Suitable technologies for assessment of variations in brushing dynamics in this way might include an inertial sensor (e.g. a multi-axial accelerometer), a force sensor, a pressure sensor (for instance, using optical pressure sensing) or an IMU able to measure dynamical motion of the support body 12 (e.g. brush head platen) in a single or, preferably, multiple directions.

The sensor means 30 which is used may be integrated in the support body, as shown in Fig. 8. If the oral care device is a toothbrush, the sensor means may be integrated in the platen of the brush head. If the oral care device is instead a mouthpiece device, it may be integrated in one or more walls of the mouthpiece device, as will be discussed further below. A communication channel may be provided between the sensor means and the processing arrangement.

With reference to Fig. 9, in one or more alternative embodiments, the sensing means 30 may comprise a pressure or force-sensitive ring member 80 arranged extending around a base of the at least one cleaning element 14, for detecting loading force or pressure applied to the cleaning element in at least one direction. The at least one direction may be a direction perpendicular to a direction of extension of the cleaning element.

By way of example, the ring member 80 may be formed of an electroactive material such as a piezoelectric material (e.g. pressure-sensitive silicone), such as an electroactive polymer. By way of further example, there may be provided sensing elements disposed on each ring member, for example deposited and printed on the ring member. For example these sensing elements may comprise strain gauges.

With reference to Fig. 9(b), in some examples, the ring member may be circumferentially segmented to provide a plurality of circumferential segments 82a, 82b, 82c, 82d which each independently generate a segment sensing signal. This permits the ring member to provide directional sensing information, based upon which of the segments are stimulated by application of pressure. The pressure sensitive ring member thus effectively acts as a force or moment sensor, i.e. a 3D dynamometer.

Although the rings are shown with four segments in the particular example of Fig. 9(b), this is clearly exemplary only and the ring member 80 may be divided into any desired number of segments, for example to segments as shown in Fig. 9(a), or may not be segmented at all.

As shown in Fig. 9, the ring members 80 are provided disposed on the surface of the support body 12. They are arranged for surrounding the base of the at least one cleaning element 14. In some examples the cleaning elements may be bristles. In some examples the cleaning elements may be arranged into tufts, in which case the rings may each be arranged to extend around the base of a tuft of cleaning elements. The reference in this disclosure to the 'at least one cleaning element" may be reference to at least one tuft of cleaning elements. In further examples, the cleaning elements may include elastomeric cleaning elements, such as silicon cleaning elements (thicker than a typical bristle), and the ring may surround such an elastomeric cleaning element.

Proposed embodiments involve sensing force, pressure or motion during use of the oral care device in a cleaning session. In such a context, the user applies a load to the cleaning elements 14 and the support body 12 as they clean their teeth. To increase the specificity of the measurements, calibration data might be used which provides an indication of the influence of user load on the tooth mobility determination, and for instance indicating the range of mobility that can be assessed for a given user pressure level.

For example, in some embodiments, the processing arrangement 20 may be adapted to perform a calibration operation comprising sensing a baseline or average loading force applied to the at least one cleaning element during a cleaning session, and wherein the degree of tooth mobility is determined in dependence upon the baseline or average loading force.

For example, the derived degree of tooth mobility may be based on a measured change in the amplitude or the width of a dominant peak in the frequency spectrum of the oscillation of the cleaning element (i.e. damping), and wherein the baseline loading force is taken into account in this computation, e.g. to normalize the detected amplitude or damping change.

The damping is proportional to the width of the dominant (i.e. resonant) peak about the peak's center frequency.

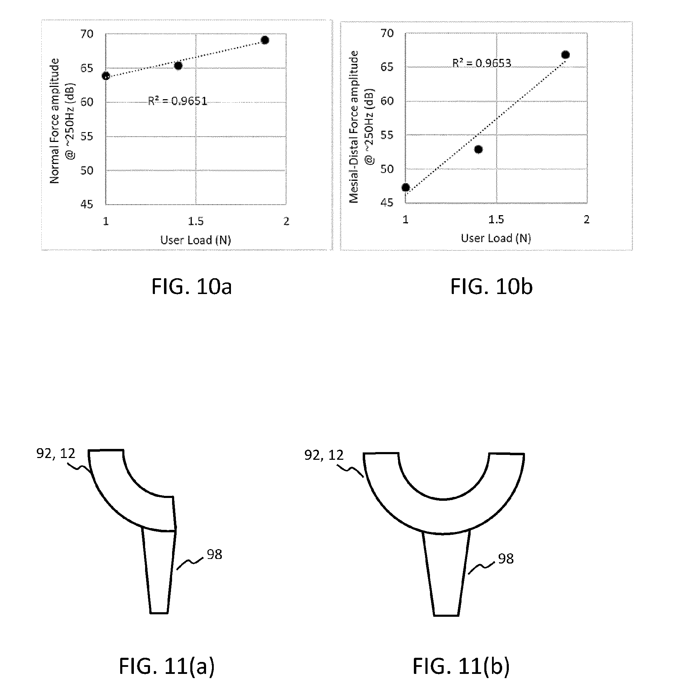

For example, reference is made to Fig. 10, which shows an example relationship between applied user load and corresponding measured normal reaction force at a single frequency component of a frequency spectrum of the force sensing signal 32 for the normal (Fig. 10(a)) and mesial-distal (Fig. 10(b)) force components. The single frequency component is that which corresponds to a dominant frequency component (i.e. main harmonic component) of the applied oscillatory motion.

Fig. 10 shows that a linear relationship (R² = 0.965) between the amplitude of the main harmonic component of the measured reaction forces (normal and mesial-distal) and the user load can be retrieved (for a constant level of tooth mobility). This can then be used to calibrate the mobility level determinations.

This could be done in different ways. For instance, a dedicated calibration procedure could be implemented. Here, the user is instructed to apply the cleaning elements to a single tooth and vary the load that they apply. The resulting measurement signal for the calibration procedure corresponds to a single tooth with a single level of mobility. The variations in the load being applied by the user can be detected for example by extracting the baseline of a force or pressure sensing signal, wherein the applied user load can be expected to be the dominant component of the baseline, and thus relative changes in the baseline map onto changes in the user load. At the same time, from the sensing signal for the calibration operation period, the amplitude of the dominant harmonic component of oscillation (e.g. 250 Hz in the example of Fig. 10) can be monitored as a function of time over this period. Then, the extracted user load signal and extracted amplitude signal can be temporally registered to one another, to thereby derive a plot of the amplitude as a function of the (relative) applied user load, and from this a linear correlation between the two (i.e. a linear mapping) derived.

This correlation can then be applied in operation to calibrate the sensing signal according to varying user load during a cleaning session, or according to an average user load derived from data acquired over one or more cleaning sessions.

Whilst embodiments mentioned above were described with reference to an oral care device in the form of a powered toothbrush for detection of a mobile tooth, a similar approach can be used for an oral care device in the form of either partial or full mouthpiece

In particular, and with reference to Fig. 11, in accordance with at least one set of embodiments, the oral care device takes the form of a mouthpiece device 90, and wherein the support body is an arcuate shaped body 92 for receipt inside the oral cavity.

With reference to Fig. 11(a), the mouthpiece 90 can be a full mouthpiece having an arcuate support body 12, 92 for following the contour of, and extending along, all or most of the dental arch of the user. This is referred to as a "C-shaped" mouthpiece. Alternatively, and with reference to Fig. 11(b), the mouthpiece may be a partial mouthpiece, having an arcuate support body 12, 92 for following the contour of only one portion of the user's dental arch. The latter design may be referred to as a "J-shaped" mouthpiece. wherein the body is moveable by a user from one side of the mouth to a second side of the mouth, and wherein the processor is adapted to acquire at least one tooth mobility metric for each side of the mouth.

In each case, the mouthpiece may optionally have a handle portion 98 attached to the arcuate support body 12, 92 portion. This aids a user in inserting and removing the mouthpiece and optionally moving it around the mouth and adjusting its positioning in the mouth. The handle portion may house the processing arrangement 20 in some examples.

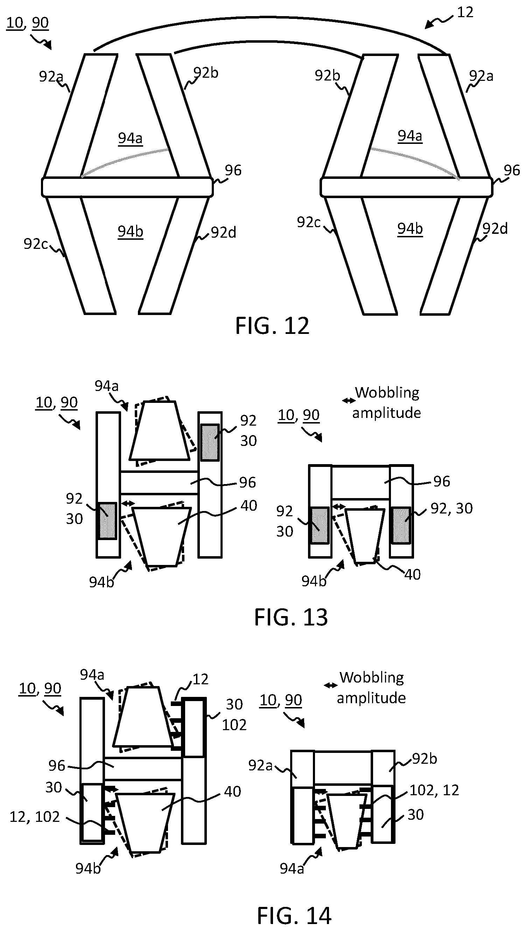

With reference to Fig. 12, in general the support body 92 of the mouthpiece device 90 defines at least one tooth-receiving channel 94, the channel bounded by respective walls 92 on either side of the channel. There may be cleaning elements provided protruding from channel-facing surfaces of the walls into the channel 94. Alternatively, the walls themselves may form the cleaning elements, providing a cleaning function through a vibration action for instance.

In the particular example shown in Fig. 12, there are two tooth-receiving channels 94a, 94b, an upper channel 94a, and a lower channel 94b, for receiving upper and lower rows of teeth respectively. The upper channel 94a is bounded by first 92a and second 92b walls and the lower 94b channel is bounded by third 92c and fourth 92d walls. The walls 92 each extend from a central platen 96 which acts to divide the upper channel 94a from the lower channel 94b.

In alternative embodiments, the mouthpiece device 90 comprises only a single tooth receiving channel 94. In these cases, the mouthpiece is often referred to as being U-shaped in cross-section. Where the mouthpiece device 90 comprises upper and lower channels 94a, 94b, it is often referred to as being H-shaped in cross-section.

With reference to Fig. 13, in some embodiments, the sensing means 30 may comprise one or more force, pressure or inertial sensors integrated in at least one wall 92 of the tooth-receiving channel(s) 94 of the mouthpiece 90 support body 12. Alternatively, the walls may themselves be formed of a pressure or force-sensitive material, such as an electroactive material, e.g. an electroactive polymer material. Thereby the walls 92 may act themselves as sensing means.

Fig 13 shows examples of respective H-shaped and U-shaped mouthpieces 90, in each case having one or more force, pressure or inertial sensors 30 integrated into one or more walls of each of the one or more tooth-receiving channels 94.

Instead of, or in addition to, being integrated in the walls 92, one or more pressure, force or inertial sensors could be integrated in the platen 96 or the handle 98, since both of these components are directly/indirectly mechanically coupled with the cleaning elements. The sensor means senses variations of acceleration/pressure/force in either the portion of the mouthpiece inserted into the mouth or the external handle, in either a direction normal to the platen 96, or preferably, in three orthogonal axes.

Detection of tooth mobility may proceed in a similar manner to that described for the powered toothbrush above. Small amplitude displacement of a mobile tooth can be detected by variations in the mouthpiece wall 92, platen 96 or handle 98 dynamics.

It would be advantageous to enable localization of a given tooth to which a sensing signal corresponds.

For an embodiment comprising a rigid full mouthpiece with a single pressure or inertia sensor, it may not be possible to exactly locate the position of the mobile tooth. However, using longitudinal measurement over multiple cleaning sessions, an indication can be given that a tooth has become (more) mobile, whereupon an indication could be given to a user, for example advising them to contact a dental professional.

In the case of a J-shaped mouthpiece, some localization of the tooth may be possible. For example, for a J-shaped mouthpiece, the arcuate support body 12 is moveable by a user from one side of the mouth to a second side of the mouth, and the processor may be adapted to acquire at least one tooth mobility metric for each side of the mouth. Again, in this design, the sensor means could be incorporated into one or more walls 92 of the mouthpiece 90 support body 12, one or more walls may be formed of a pressure or force sensitive material to thereby perform the function of the sensing means.

It is possible to provide localization information related to the acquired sensing signal by, for example comparing the sensor signals for the left and the right-hand side of the mouth (in the case where the user first brushes one side of the mouth and subsequently inverts the mouthpiece and then covers the other side). In the case where the user moves the mouthpiece slowly around the arch of the teeth, the processing arrangement may be adapted to dynamically monitor the signal and generate a user alert when a (more) mobile tooth is, and/or indicating when a (more) mobile tooth signal is no longer being sensed, as the mouthpiece moves off of the relevant tooth.

With reference to Fig. 14, in some embodiments, cleaning elements may be provided 102 extending into the tooth receiving channel for engagement with teeth, and wherein the at least one cleaning element 102 comprises a pressure-responsive material for sensing force or pressure applied to the cleaning element, and wherein the pressure-responsive material forms at least a part of the sensing means 30.

This thereby provides a variant of the above-described embodiments. The tooth wobbling, typically expressed by characteristic dynamic transient force signals and force signatures, can be measured using a mouthpiece comprising integrated electroactive cleaning elements 102 (e.g. conductive cleaning elements) acting as a dynamic force sensor.

The electroactive cleaning elements 102 extend into the tooth-receiving channel 94 from one or both walls 92 of the channel. The electroactive cleaning elements act as force or pressure sensors, by outputting a sensing signal correlated with pressure or force applied by the tooth to the elements. For example the elements 102 are formed of an electroactive material such an electroactive polymer material or piezoelectric material, or may be formed of a conductive material and wherein deformation of the elements by applied pressure from the tooth changes an electrical property in the element such as resistance or capacitance in a way that is detectable by the operatively coupled processing arrangement 20.

In some embodiments, the mouthpiece can be made of a series of individually driven platens (for example connected by flexible sections). In such examples, a multiplicity of force, pressure or inertia sensors could be incorporated (for example one sensor for each platen) to assist in localization by indicating in which section a signal for a mobile tooth is present. Again, comparing signals from left and right sides of the mouth may further help to improve mobile tooth detection.

In other words, in these particular examples, the support body 92 comprises a plurality of sections and each comprising a respective sensor for sensing force on, or motion of, at least one cleaning element protruding from the respective section, and wherein a respective tooth mobility measure is determined for each section.

In these particular examples, each section of the body may be vibrationally insulated from each neighboring section; and/or each section of the body is independently drivable with an oscillatory motion.

Embodiments proposed herein enable home-based tooth mobility assessment while brushing using consumer oral cleaning devices. Proposed embodiments enable methods or algorithms for triaging of oral care diseases and conditions, such as periodontal disease, bruxism, malocclusion, and monitoring of orthodontic treatments, based on monitoring changes in tooth mobility level or degree over time.

Another aspect of the description provides a computer-implemented method comprising:
receiving a sensing signal from a sensor means 30 corresponding to a period of mechanical engagement of at least one cleaning element 14 or an oral care device with at least one tooth 40 during an oral cleaning session, wherein the sensing signal provides a measure indicative of a loading force applied to, or motion of, at least one of the cleaning elements 14;
processing a waveform 60 of the signal 32 to extract one or more characteristics of the signal waveform for the time period;
determining a measure indicative of a degree of tooth mobility 26 for the at least one tooth 40; and
generating a data signal 25 indicative of the determined measure indicative of a degree of tooth mobility.

The oral care device from which the signal is received may comprise a support body 12; and a plurality of cleaning elements 14 for mechanical engagement with oral surfaces 42, the cleaning elements protruding from a surface of the support body.

Another aspect of the description is a computer program product comprising code means configured, when run on a processor, to cause the processor to perform a computer implemented method in accordance with the method outlined above.

Features of the apparatus may be combined with the method or the computer program product.

Embodiments of the invention described above employ a processing arrangement. The processing arrangement may in general comprise a single processor or a plurality of processors. It may be located in a single containing device, structure or unit, or it may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors of the processing arrangement can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

The scope of protection is determined by the claims.

## Claims

1. An oral care device (10), comprising:
a support body (12);
a plurality of cleaning elements (14) for mechanical engagement with oral surfaces (42), the cleaning elements protruding from a surface of the support body;
a sensor means (30) adapted to sense a measure indicative of a loading force or pressure applied to, or motion of, at least one of the cleaning elements (14); and
a processing arrangement (20) comprising one or more processors (24) adapted to:
receive a sensing signal (32) from the sensor means (30) corresponding to a period of mechanical engagement of the at least one cleaning element (14) with at least one tooth (40) during an oral cleaning session,
process a waveform (60) of the signal (32) to extract one or more characteristics of the signal waveform for the time period;
determine a measure indicative of a degree of tooth mobility (26) for the at least one tooth (40) from the one or more characteristics; and
generate a data signal (25) indicative of the determined measure indicative of a degree of tooth mobility, for output from the device.

2. A device (10) as claimed in claim 1, wherein determining the degree of tooth mobility comprises performing a spectral analysis of the sensing signal.

3. A device (10) as claimed in claim 2, wherein determining the degree of tooth mobility comprises computing a frequency spectrum (70, 72) for the sensing signal, and
detecting an amplitude of one or more frequency components of the sensing signal; and/or
detecting a bandwidth or a quality factor associated with a dominant peak in the frequency spectrum.

4. A device (10) as claimed in claim 3, wherein the oral care device includes an actuation means (19) for driving an oscillatory motion of the at least one cleaning element (14) which, in a reference condition, exhibits a reference oscillatory frequency spectrum having a dominant peak therein, and wherein the determining the degree of tooth mobility comprises determining changes between the reference frequency spectrum and the sensed frequency spectrum of
- an amplitude of a central frequency of the dominant peak of the frequency spectrum;
- a bandwidth associated with the dominant peak of the frequency spectrum; and/or
- a quality factor associated with the dominant peak of the frequency spectrum.

5. A device as claimed in claim 1, wherein the processing arrangement is adapted to perform a calibration operation comprising sensing a baseline or average loading force applied to the at least one cleaning element during at least one cleaning session, and wherein the degree of tooth mobility is determined in dependence upon the baseline or average loading force.

6. A device (10) as claimed in any of claims 1-5, wherein the oral care device is a toothbrush with a brush head (16) coupled to a main body (18), wherein the main body forms a handle for the device, and wherein the support body (12) is a platen of the brush head, and preferably wherein the cleaning elements (14) comprise bristles.

7. A device (10) as claimed in claim 6, wherein the sensor means (30) comprises a force or pressure sensor (52) adapted to detect loading force applied to the brush head relative to the main body.

8. A device as claimed in any of claims 1-6, wherein the sensor means (30) comprises a pressure-sensitive ring member (80) extending around a base of the at least one cleaning element (14), for detecting loading pressure applied to the cleaning element in at least one direction perpendicular to a direction of extension of the cleaning element, and
optionally wherein the pressure-sensitive ring member (80) comprises a pressure-responsive electroactive material.

9. A device (10) as claimed in any of claims 1-8, wherein the oral care device is a mouthpiece device (90), and wherein the support body (92, 96) is for receipt inside the oral cavity.

10. A device (10) as claimed in claim 9, wherein the support body (92, 96) defines at least one tooth-receiving channel (94a, 94b), and wherein:
the sensor means (30) comprises one or more force or pressure sensors integrated in at least one wall (92) of the tooth-receiving channel (94); and/or
the cleaning elements (12) extend into the tooth receiving channel for engagement with teeth, and wherein the at least one cleaning element comprises a pressure-responsive material for sensing force or pressure applied to the cleaning element, and wherein the pressure-responsive material forms at least a part of the sensor means (30, 102).

11. A device as claimed in claim 9 or 10, wherein the support body (92) comprises a J-shaped arcuate body for following the contour of one portion of the user's dental arch, and wherein the body is moveable by a user from one side of the mouth to a second side of the mouth, and wherein the processing arrangement is adapted to acquire at least one tooth mobility metric for each side of the mouth.

12. A device as claimed in claim 9 or 10, wherein the support body (92) comprises a plurality of sections (92a, 92b), and each comprising a respective sensor for sensing force on, or motion of, at least one cleaning element protruding from the respective section, and wherein a respective tooth mobility measure is determined for each section.

13. A device as claimed in any of claims 1-12, wherein the processing arrangement is further adapted to:
- determine a degree of tooth mobility for each of a plurality of teeth in the oral cavity over the course of an oral cleaning session, to thereby generate a tooth mobility data record for the oral cleaning session; and
- transfer the tooth mobility data record to a datastore (28), tagged with a time-stamp, and optionally tagged with user identification information.

14. A system (8) comprising:
a device (10) in accordance with any of claims 1-13;
a datastore (28) for storing previously determined metrics of tooth mobility for one or more subjects; and
a secondary device (112) comprising a processing arrangement (104) adapted to:
access the datastore (28);
retrieve multiple tooth mobility data records for a given subject for a series of different time points, each record comprising tooth mobility metrics for one or more teeth; and
control a user interface (106) of the secondary device to display trend information indicative of the one or more tooth mobility metrics as a function of time.

## Patentansprüche

1. Mundpflegevorrichtung (10), umfassend:
einen Trägerkörper (12);
eine Vielzahl von Reinigungselementen (14) für mechanischen Kontakt mit Mundoberflächen (42), wobei die Reinigungselemente von einer Oberfläche des Trägerkörpers vorstehen;
ein Sensormittel (30), welches dazu adaptiert ist, einen Messwert zu erfassen, welcher auf eine auf zumindest eines der Reinigungselemente (14) ausgeübte Belastungskraft oder einen Druck oder auf dessen Bewegung hinweist; und
eine Verarbeitungsanordnung (20), welche einen oder mehrere Prozessoren (24) umfasst, welche dazu adaptiert sind:
ein Messsignal (32) von dem Sensormittel (30) zu empfangen, welches einer Dauer von mechanischem Kontakt des zumindest einen Reinigungselements (14) mit zumindest einem Zahn (40) während einer Mundreinigungssitzung entspricht,
eine Wellenform (60) des Signals (32) zu verarbeiten, um eine oder mehrere Eigenschaften der Signalwellenform für die Zeitdauer zu extrahieren;
aus einer oder mehreren Eigenschaften einen Messwert zu bestimmen, welcher auf einen Grad der Zahnbeweglichkeit (26) des zumindest einen Zahnes (40) hinweist; und
ein Datensignal (25), welches auf den bestimmten Messwert hinweist, welcher auf einen Grad der Zahnbeweglichkeit hinweist, zur Ausgabe durch die Vorrichtung zu erzeugen.

2. Vorrichtung (10) wie in Anspruch 1 beansprucht, wobei Bestimmen des Grads der Zahnbeweglichkeit Durchführen einer Spektralanalyse des Messsignals umfasst.

3. Vorrichtung (10) wie in Anspruch 2 beansprucht, wobei Bestimmen des Grads der Zahnbeweglichkeit Berechnen eines Frequenzspektrums (70, 72) für das Messsignal umfasst, und
Erkennen einer Amplitude einer oder mehrerer Frequenzkomponenten des Messsignals; und/oder
Erkennen einer Bandbreite oder eines Gütefaktors, welcher mit einer dominierenden Spitze in dem Frequenzspektrum verknüpft ist.

4. Vorrichtung (10) wie in Anspruch 3 beansprucht, wobei die Mundpflegevorrichtung ein Betätigungsmittel (19) zum Antreiben einer Schwingbewegung des zumindest einen Reinigungselements (14) beinhaltet, welche in einem Referenzzustand ein Referenz-Schwingfrequenzspektrum mit einer dominierenden Spitze aufweist, und wobei das Bestimmen des Grads der Zahnbeweglichkeit Bestimmen von Änderungen zwischen dem Referenzfrequenzspektrum und dem erfassten Frequenzspektrum umfasst, nämlich
- einer Amplitude einer Mittenfrequenz der dominierenden Spitze des Frequenzspektrums;
- einer Bandbreite, welche mit der dominierenden Spitze des Frequenzspektrums verknüpft ist; und/oder
- eines Gütefaktors, welcher mit der dominierenden Spitze des Frequenzspektrums verknüpft ist.

5. Vorrichtung wie in Anspruch 1 beansprucht, wobei die Verarbeitungsanordnung dazu adaptiert ist, einen Kalibrierungsvorgang durchzuführen, welcher Erfassen einer grundlegenden oder durchschnittlichen Belastungskraft umfasst, welche während zumindest einer Reinigungssitzung auf das zumindest eine Reinigungselement ausgeübt wird, und wobei der Grad der Zahnbeweglichkeit in Abhängigkeit von der grundlegenden oder durchschnittlichen Belastungskraft bestimmt wird.

6. Vorrichtung (10) nach einem der Ansprüche 1-5, wobei es sich bei der Mundpflegevorrichtung um eine Zahnbürste mit einem Bürstenkopf (16) handelt, welcher mit einem Hauptkörper (18) verbunden ist, wobei der Hauptkörper einen Griff für die Vorrichtung bildet, und wobei der Trägerkörper (12) eine Platte des Bürstenkopfes ist, und wobei bevorzugt die Reinigungselemente (14) Borsten umfassen.

7. Vorrichtung (10) wie in Anspruch 6 beansprucht, wobei das Sensormittel (30) einen Kraft- oder Drucksensor (52) umfasst, welcher dazu adaptiert ist, die auf den Bürstenkopf relativ zu dem Hauptkörper ausgeübte Belastungskraft zu erkennen.

8. Vorrichtung wie in einem der Ansprüche 1-6 beansprucht, wobei das Sensormittel (30) ein druckempfindliches Ringelement (80) umfasst, welches sich um eine Basis des zumindest einen Reinigungselements (14) erstreckt, um den auf das Reinigungselement in zumindest einer Richtung senkrecht zu der Richtung der Erstreckung des Reinigungselements ausgeübten Belastungsdruck zu erkennen, und
wobei optional das druckempfindliche Ringelement (80) ein druckabhängiges elektroaktives Material umfasst.

9. Vorrichtung (10) wie in einem der Ansprüche 1-8 beansprucht, wobei es sich bei der Mundpflegevorrichtung um eine Mundstückvorrichtung (90) handelt, und wobei der Trägerkörper (92, 96) zur Aufnahme in der Mundhöhle bestimmt ist.

10. Vorrichtung (10) wie in Anspruch 9 beansprucht, wobei der Trägerkörper (92, 96) zumindest einen Zahnaufnahmekanal (94a, 94b) definiert, und wobei:
das Sensormittel (30) einen oder mehrere Kraft- oder Drucksensoren umfasst, welche in zumindest einer Wand (92) des Zahnaufnahmekanals (94) integriert sind; und/oder
die Reinigungselemente (12) sich in den Zahnaufnahmekanal hinein erstrecken, um mit Zähnen in Kontakt zu kommen, und wobei das zumindest eine Reinigungselement ein druckabhängiges Material zum Erfassen der auf das Reinigungselement ausgeübten Kraft oder des ausgeübten Drucks umfasst, und wobei das druckabhängige Material zumindest ein Teil der Sensormittel (30, 102) bildet.

11. Vorrichtung wie in Anspruch 9 oder 10 beansprucht, wobei der Trägerkörper (92) einen J-förmigen bogenförmigen Körper umfasst, um der Kontur eines Abschnitts des Zahnbogens des Benutzers zu folgen, und wobei der Körper durch einen Benutzer von einer Seite des Mundes zu einer zweiten Seite des Mundes beweglich ist, und wobei die Verarbeitungsanordnung dazu adaptiert ist, zumindest eine Metrik der Zahnbeweglichkeit für jede Seite des Mundes zu gewinnen.

12. Vorrichtung wie in Anspruch 9 oder 10 beansprucht, wobei der Trägerkörper (92) eine Vielzahl von Bereichen (92a, 92b) umfasst, und jeweils einen Sensor zum Erfassen von Kraft auf ein oder Bewegung zumindest eines aus dem jeweiligen Bereich vorstehenden Reinigungselements umfasst, und wobei für jeden Bereich ein entsprechender Messwert für die Zahnbeweglichkeit bestimmt wird.

13. Vorrichtung wie in einem der Ansprüche 1-12 beansprucht, wobei die Verarbeitungsanordnung weiter dazu adaptiert ist:
- einen Grad der Zahnbeweglichkeit für jeden einzelnen Zahn einer Vielzahl von Zähnen in der Mundhöhle im Verlauf einer Mundreinigungssitzung zu bestimmen, um dadurch einen Datensatz zur Zahnbeweglichkeit für die Mundreinigungssitzung zu erzeugen; und
- den Datensatz zur Zahnbeweglichkeit mit einem Zeitstempel und optional mit Benutzeridentifikationsinformationen versehen in einen Datenspeicher (28) zu übertragen.

14. System (8) umfassend:
eine Vorrichtung (10) gemäß einem der Ansprüche 1-13;
einen Datenspeicher (28) zum Speichern zuvor bestimmter Metriken der Zahnbeweglichkeit für einen oder mehrere Probanden; und
eine sekundäre Vorrichtung (112), welche eine Verarbeitungsanordnung (104) umfasst, welche dazu adaptiert ist:
auf den Datenspeicher (28) zuzugreifen;
mehrere Datensätze zur Zahnbeweglichkeit für einen bestimmten Probanden in einer Abfolge verschiedener Zeitpunkte abzurufen, wobei jeder Datensatz Metriken zur Zahnbeweglichkeit für einen oder mehrere Zähne umfasst; und
eine Benutzeroberfläche (106) der sekundären Vorrichtung zu steuern, um Trendinformationen anzuzeigen, welche auf die eine oder mehreren Metriken zur Zahnbeweglichkeit als eine Funktion der Zeit hinweisen.

## Revendications

1. Dispositif de soin buccal (10), comprenant :
un corps de support (12) ;
une pluralité d'éléments de nettoyage (14) pour l'engagement mécanique avec les surfaces orales (42), les éléments de nettoyage faisant saillie d'une surface du corps de support ;
un moyen de capteur (30) adapté pour détecter une mesure indicative d'une force de charge ou d'une pression appliquée à, ou d'un mouvement de, au moins un des éléments de nettoyage (14) ; et
un agencement de traitement (20) comprenant un ou plusieurs processeurs (24) adapté pour :
recevoir un signal de détection (32) provenant du moyen de capteur (30) correspondant à une période d'engagement mécanique du au moins un élément de nettoyage (14) avec au moins une dent (40) au cours d'une séance de nettoyage buccal,
traiter une forme d'onde (60) du signal (32) pour extraire une ou plusieurs caractéristiques de la forme d'onde de signal pour la période de temps ;
déterminer une mesure indicative d'un degré de mobilité dentaire (26) pour la au moins une dent (40) à partir des une ou plusieurs caractéristiques ; et
générer un signal de données (25) indicatif de la mesure déterminée indiquant un degré de mobilité dentaire, pour la sortie du dispositif.

2. Dispositif (10) selon la revendication 1, dans lequel la détermination du degré de mobilité dentaire comprend l'exécution d'une analyse spectrale du signal de détection.

3. Dispositif (10) selon la revendication 2, dans lequel la détermination du degré de mobilité dentaire comprend le calcul d'un spectre de fréquences (70, 72) pour le signal de détection, et
la détection d'une amplitude d'une ou plusieurs composantes de fréquence du signal de détection ; et/ou
la détection d'une bande passante ou d'un facteur de qualité associé à un pic dominant dans le spectre de fréquences.

4. Dispositif (10) selon la revendication 3, dans lequel le dispositif de soin buccal inclut un moyen d'actionnement (19) pour entraîner un mouvement oscillatoire du au moins un élément de nettoyage (14) qui, dans une condition de référence, présente un spectre de fréquences oscillatoires de référence présentant un pic dominant dans celui-ci, et dans lequel la détermination du degré de mobilité dentaire comprend la détermination de variations entre le spectre de fréquences de référence et le spectre de fréquences détecté de
- l'amplitude d'une fréquence centrale du pic dominant du spectre de fréquences ;
- une bande passante associée au pic dominant du spectre de fréquences ; et/ou
- un facteur de qualité associé au pic dominant du spectre de fréquences.

5. Dispositif selon la revendication 1, dans lequel l'agencement de traitement est adapté pour effectuer une opération d'étalonnage comprenant la détection d'une force de charge de base ou moyenne appliquée à au moins un élément de nettoyage au cours d'au moins une session de nettoyage, et dans lequel le degré de mobilité dentaire est déterminé en fonction de la force de charge de base ou moyenne.

6. Dispositif (10) selon l'une quelconque des revendications 1-5, dans lequel le dispositif de soin buccal est une brosse à dents avec une tête de brosse (16) couplée à un corps principal (18), dans lequel le corps principal forme une poignée pour le dispositif, et dans lequel le corps de support (12) est un plateau de la tête de brosse, et de préférence dans lequel les éléments de nettoyage (14) comprennent des poils.

7. Dispositif (10) selon la revendication 6, dans lequel le moyen de capteur (30) comprend un capteur de force ou de pression (52) adapté pour détecter la force de charge appliquée à la tête de brosse par rapport au corps principal.

8. Dispositif selon l'une quelconque des revendications 1-6, dans lequel le moyen de capteur (30) comprend un élément annulaire sensible à la pression (80) s'étendant autour de la base du au moins un élément de nettoyage (14), pour détecter la pression de charge appliquée à l'élément de nettoyage dans au moins une direction perpendiculaire à la direction d'extension de l'élément de nettoyage, et
facultativement dans lequel l'élément annulaire sensible à la pression (80) comprend un matériau électroactif sensible à la pression.

9. Dispositif (10) selon l'une quelconque des revendications 1-8, dans lequel le dispositif de soin buccal est un dispositif d'embout buccal (90), et dans lequel le corps de support (92, 96) est destiné à être reçu à l'intérieur de la cavité buccale.

10. Dispositif (10) selon la revendication 9, dans lequel le corps de support (92, 96) définit au moins un canal de réception de dent (94a, 94b), et dans lequel :
le moyen de capteur (30) comprend un ou plusieurs capteurs de force ou de pression intégrés dans au moins une paroi (92) du canal de réception de dent (94) ; et/ou
les éléments de nettoyage (12) s'étendent dans le canal de réception de dent pour s'engager avec les dents, et dans lequel le au moins un élément de nettoyage comprend un matériau sensible à la pression pour détecter la force ou la pression appliquée à l'élément de nettoyage, et dans lequel le matériau sensible à la pression forme au moins une partie du moyen de capteur (30, 102).

11. Dispositif selon la revendication 9 ou 10, dans lequel le corps de support (92) comprend un corps arqué en forme de J destiné à suivre le contour d'une partie de l'arcade dentaire de l'utilisateur, et dans lequel le corps peut être déplacé par l'utilisateur d'un côté de la bouche à un second côté de la bouche, et dans lequel l'agencement de traitement est adapté pour acquérir au moins une mesure de mobilité dentaire pour chaque côté de la bouche.

12. Dispositif selon la revendication 9 ou 10, dans lequel le corps de support (92) comprend une pluralité de sections (92a, 92b), et chacune comprenant un capteur respectif pour détecter la force sur ou le mouvement d'au moins un élément de nettoyage faisant saillie de la section respective, et dans lequel une mesure de mobilité dentaire respective est déterminée pour chaque section.

13. Dispositif selon l'une quelconque des revendications 1-12, dans lequel l'agencement de traitement est en outre adapté pour :
- déterminer le degré de mobilité dentaire de chacune d'une pluralité de dents de la cavité buccale au cours d'une séance de nettoyage buccal, afin de générer un enregistrement de données de mobilité dentaire pour la séance de nettoyage buccal ; et
- transférer l'enregistrement de données de mobilité dentaire vers un magasin de données (28), étiqueté avec un horodatage et facultativement étiqueté avec des informations d'identification de l'utilisateur.

14. Système (8) comprenant :
un dispositif (10) conforme à l'une quelconque des revendications 1-13 ;
un magasin de données (28) pour stocker des mesures de mobilité dentaire préalablement déterminées pour un ou plusieurs sujets ; et
un dispositif secondaire (112) comprenant un agencement de traitement (104) adapté pour :
accéder au magasin de données (28) ;
récupérer de multiples enregistrements de données de mobilité dentaire d'un sujet donné pour une série de moments différents, chaque enregistrement comprenant des mesures de mobilité dentaire pour une ou plusieurs dents ; et commander une interface utilisateur (106) du dispositif secondaire pour afficher des informations de tendance indicatives des une ou plusieurs mesures de mobilité dentaire en fonction du temps.
